# EUROPEAN PATENT APPLICATION

(11) **EP 4 830 710 A1**
(43) Date of publication of application: **28.10.2026**
(21) Application number: 24908175.3
(22) Date of filing: 20.12.2024
(51) Int. Cl.: A61K 35/28, A61K 9/50, A61P 25/00, A61P 9/10

(54) **METHOD FOR PREPARING EXTRACELLULAR VESICLES DERIVED FROM GLIA-LIKE CELLS DIFFERENTIATED FROM STEM CELLS, AND COMPOSITION FOR ALLEVIATING OR TREATING NERVE DAMAGE, COMPRISING SAME AS ACTIVE INGREDIENT**

(30) Priority: 22.12.2023 KR 20230189772; 06.12.2024 KR 20240180722
(71) Applicant: Seoul National University R&DB Foundation, Seoul 08826 (KR); Konkuk University Industrial Cooperation Corp, Seoul 05029 (KR)
(72) Inventor: CHO, Ssang Goo, Seoul 06597 (KR); CHANG, Mi Sook, Seoul 03080 (KR)
(74) Representative: Maiwald GmbH
(86) International application number: PCT/KR2024/020850
(87) International publication number: WO 2025/135893

(57) **Abstract**

The present invention relates to a method for preparing extracellular vesicles derived from glia-like cells differentiated from stem cells, and a composition for preventing or treating nervous system diseases caused by nerve damage, comprising same as an active ingredient. The extracellular vesicles, of the present invention, which are separated after pretreating, with a specific peptide, the glia-like cells differentiated from stem cells, have neuroprotective and regenerative effects, and thus can be effectively used for preventing or treating nervous system diseases caused by nerve damage. In addition, the method for preparing extracellular vesicles, of the present invention, can produce, in a high yield, stem cell-derived extracellular vesicles having a nerve regeneration effect, and thus is useful.

## Description

### Technical Field

The present invention relates to a method for preparing extracellular vesicles derived from glia-like cells differentiated from stem cells, and a composition for preventing or treating neurological disease caused by nerve injury, comprising same as an active ingredient.

### Related Art

Neuropathy caused by nerve injury resulting from diabetes, inflammation, anticancer agents, trauma, and the like causes severe chronic pain and motor dysfunction.

For a long period of time, studies have been conducted to inhibit or alleviate pain or permanent functional paralysis caused by nerve injury.

To date, various potential pharmacological treatments, including steroids, antioxidants, glutamate receptor inhibitors, ion channel inhibitors, gangliosides, antibodies against axonal regeneration inhibitors, anti-inflammatory agents, and neurotrophic factors, have been attempted. However, these drugs have many problems, such as unclear therapeutic efficacy and side effects caused by excessive administration.

In addition, tricyclic antidepressants, anticonvulsants, serotonin and noradrenaline reuptake inhibitors, and opioids, which are known as drugs capable of suppressing pain, have been used for the purpose of reducing pain caused by peripheral neuropathy. These drugs cause various side effects and merely suppress symptoms, and no means for treating damaged nerves, which are the underlying cause, has been known to date.

As described above, treatments for nerve injury using drugs or physiotherapy have reached their limitations, and many experimental studies have been conducted focusing on treatments using stem cells or secretions derived therefrom.

Recently, studies have reported that secretomes contain various bioactive factors capable of regulating cellular behavior. In particular, secretomes contain "exosomes" or "extracellular vesicles" having intercellular signaling functions, and studies on the components and functions thereof are being actively conducted.

In addition, extracellular vesicles reflect the state of the cells from which they are secreted (donor cells), exhibit various biological activities depending on the type of cells from which they are secreted, and play important roles in intercellular interactions by transferring genetic materials and proteins between cells.

Furthermore, extracellular vesicles exhibit lower immunogenic rejection and a lower risk of tumor formation than stem cells, thereby providing enhanced safety. In addition, extracellular vesicles are much easier to store and transport than stem cells and are capable of crossing the blood-brain barrier, which cells cannot pass through. Therefore, extracellular vesicles are highly effective for the treatment of nerve injury and have the advantage of causing fewer systemic side effects.

Moreover, therapeutic methods using extracellular vesicles have attracted attention as a novel approach capable of overcoming the disadvantages of conventional cell therapeutics, such as difficulties in securing and maintaining therapeutically effective amounts of cells, because such methods do not employ cells themselves.

Accordingly, the present inventors have made intensive efforts to study stem cell-derived extracellular vesicles, which contain various beneficial components and are composed of a lipid bilayer, thereby functioning as a stable drug delivery system by themselves. Specifically, the present inventors sought to develop extracellular vesicles having the same inherent effects as mesenchymal stem cells(hereinafter referred to as glia-like human mesenchymal stem cell, ghMSC) whose neuroprotective and regenerative efficacy is enhanced by their ability to secrete large amounts of glial growth factors. In addition, since the number of extracellular vesicles generally secreted by nucleated cells is only about 500 to 1,000 vesicles per cell, the present inventors made efforts to develop an efficient method for obtaining the extracellular vesicles.

As a result, the present inventors confirmed that, when the glia-like cells are pretreated with a human protein-derived peptide, extracellular vesicles retaining the intrinsic efficacy of the glia-like cells, i.e., mesenchymal stem cells having enhanced neuroprotective and regenerative efficacy, can be obtained with high purity and high yield, thereby completing the present invention.

### [Prior Art Documents]

### [Patent Documents]

(Patent Document 001) KR 10-2024-0032613 A

### SUMMARY OF THE INVENTION

### TECHNICAL PROBLEM

An object of the present invention is to provide a pharmaceutical composition for preventing or treating neurological diseases caused by nerve injury.

Another object of the present invention is to provide a method for preparing stem cell-derived extracellular vesicles having improved neural regenerative efficacy and yield.

### TECHNICAL SOLUTION

In order to achieve the above object, the present invention provides a pharmaceutical composition for preventing or treating a neurological disease caused by nerve injury, comprising an extracellular vesicle derived from a glia-like cell (ghMSC) differentiated from human mesenchymal stem cell (hMSC) as an active ingredient, wherein the glia-like cells are pretreated with a peptide comprising the amino acid sequence of SEQ ID NO: 1.

According to one preferred embodiment of the present invention, the mesenchymal stem cells may be bone marrow-derived mesenchymal stem cells.

According to one preferred embodiment of the present invention, the extracellular vesicle may be obtained by a method comprising the following steps:
a) differentiating a human bone marrow-derived mesenchymal stem cell to obtain a glia-like cell;
b) culturing the glia-like cell in a medium composition comprising a peptide comprising the amino acid sequence of SEQ ID NO: 1; and
c) collecting the medium composition of step b) comprising the extracellular vesicle and removing the stem cells through centrifugation.

According to one preferred embodiment of the present invention, the extracellular vesicles may have an average diameter of 50 to 300 nm.

According to one preferred embodiment of the present invention, the composition may comprise the extracellular vesicles at a concentration of 10⁶ to 10¹⁴ particles/mL.

According to one preferred embodiment of the present invention, the neurological disease caused by nerve injury is a neurological disease caused by nerve injury of the central nervous system or nerve injury of the peripheral nervous system.

According to one preferred embodiment of the present invention, the neurological disease caused by nerve injury of the central nervous system may be at least one selected from the group consisting of organic diseases and functional disorders of the central nervous system, epilepsy, multiple sclerosis, amyotrophic lateral sclerosis (ALS, Lou Gehrig's disease), Alzheimer's disease, Lewy body dementia, Huntington's disease, Parkinson's disease, schizophrenia, traumatic brain injury, stroke, Pick's disease, Creutzfeldt-Jakob disease, progressive supranuclear palsy, multiple system atrophy, corticobasal degeneration, spinocerebellar degeneration, cerebellar atrophy, post-traumatic stress disorder, amnesia, vascular dementia, and cerebral infarction.

According to one preferred embodiment of the present invention, the neurological disease caused by nerve injury of the peripheral nervous system may be at least one selected from the group consisting of peripheral neuropathy, diabetic neuropathy, peripheral neuropathic pain, chemotherapy-induced peripheral neuropathy, complex regional pain syndrome, optic neuropathy, mononeuropathy, multiple mononeuropathy (mononeuritis multiplex), polyneuropathy, Guillain-Barré syndrome (acute inflammatory demyelinating polyneuropathy), chronic inflammatory demyelinating polyneuropathy, hereditary neuropathy, plexopathy, glaucoma, macular degeneration, amyotrophic lateral sclerosis (ALS, Lou Gehrig's disease), progressive muscular atrophy, progressive bulbar palsy, poliomyelitis, post-polio syndrome, stiff-person syndrome, Isaacs' syndrome, myasthenia gravis, neonatal myasthenia, botulism, Eaton-Lambert syndrome, thoracic outlet syndrome, Charcot-Marie-Tooth disease, and spinal muscular atrophy.

In order to achieve another object of the present invention, the present invention provides a method for preparing a stem cell-derived extracellular vesicle having improved nerve regeneration efficacy and yield, comprising the following steps:
a) differentiating a human bone marrow-derived mesenchymal stem cell to obtain a glia-like cell (ghMSC);
b) culturing the glia-like cell in a medium composition comprising a peptide comprising the amino acid sequence of SEQ ID NO: 1; and
c) collecting the medium composition of step b) comprising the extracellular vesicle and removing the glia-like cell through centrifugation.

### ADVANTAGEOUS EFFECTS

The extracellular vesicles isolated from glia-like cells differentiated from stem cells and pretreated with a specific peptide according to the present invention possess neuroprotective and regenerative efficacy, and therefore can be advantageously used for the prevention or treatment of neurological diseases caused by nerve injury.

In addition, the method for preparing extracellular vesicles according to the present invention is advantageous in that stem cell-derived extracellular vesicles having neural regenerative efficacy can be produced at a high yield.

### BRIEF DESCRIPTION OF THE DRAWINGS

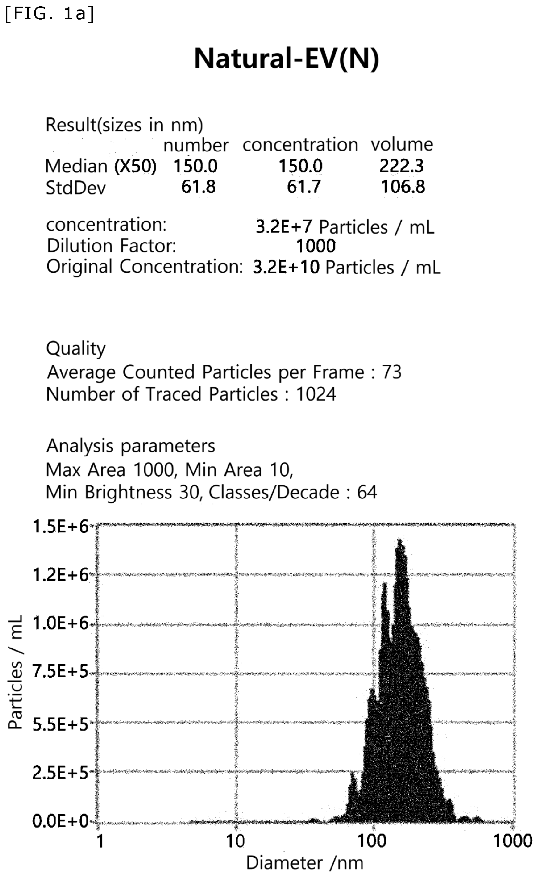

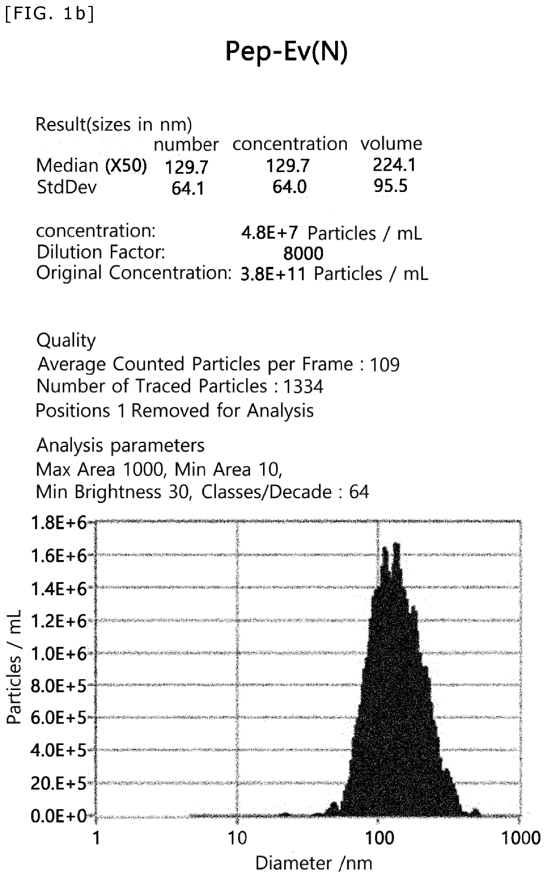

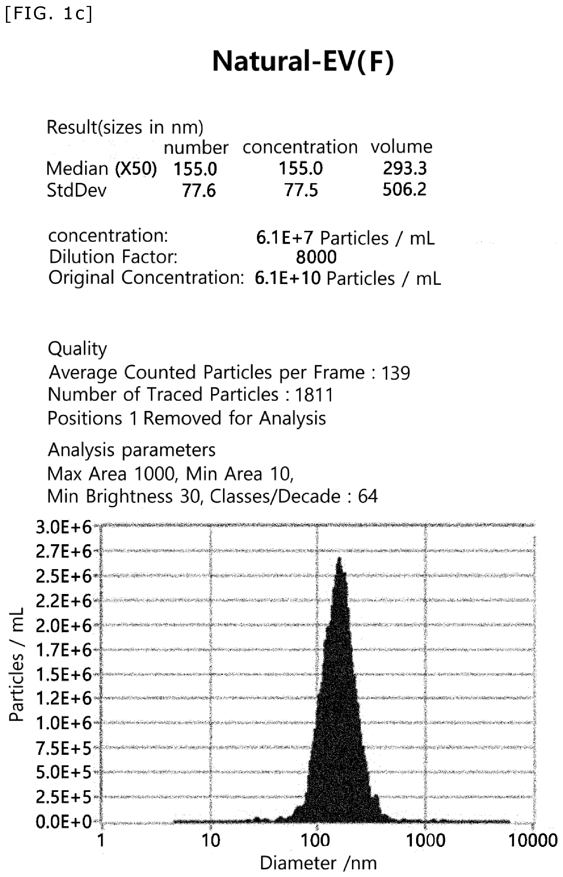

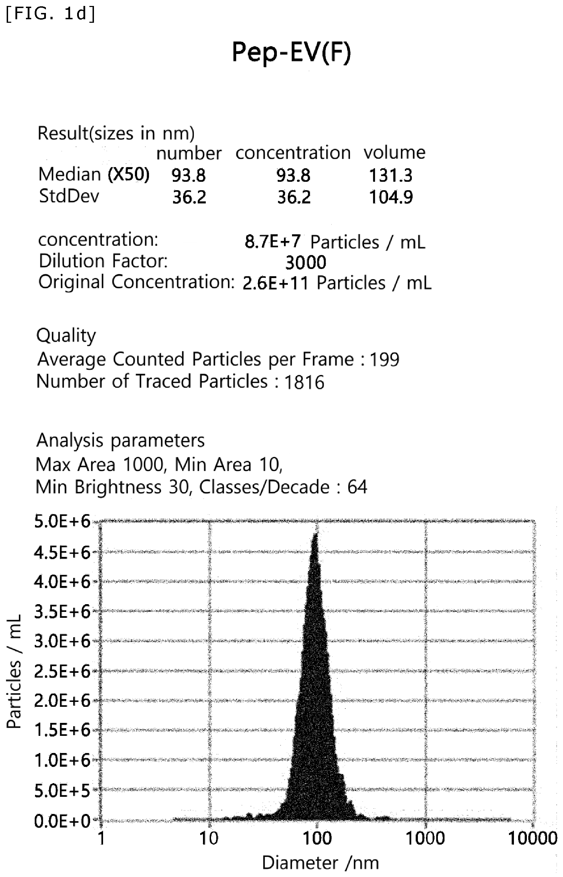

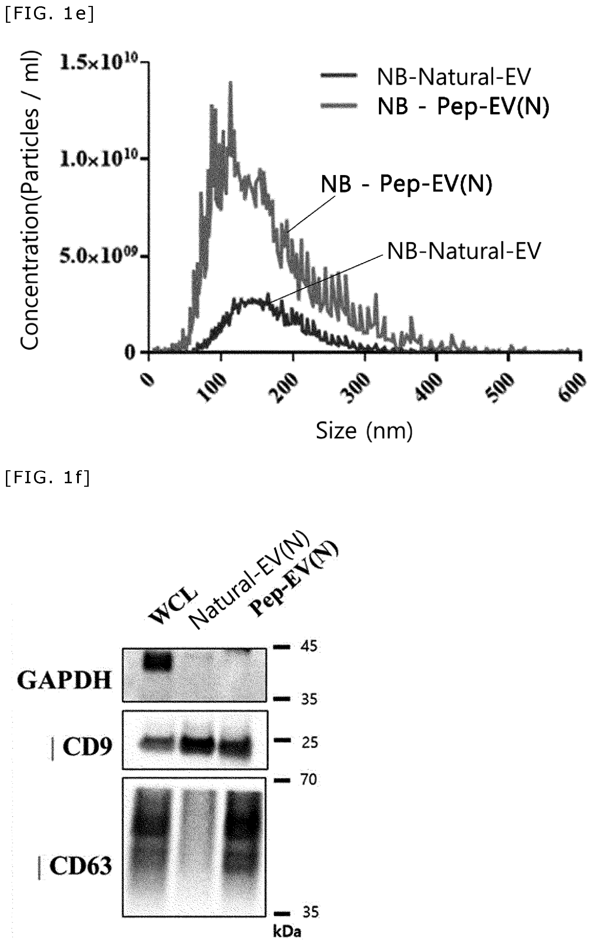

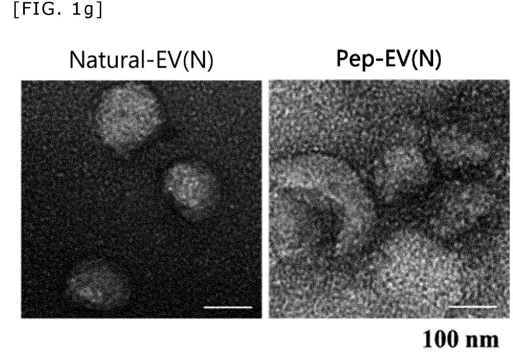

FIG. 1 shows the results of analyzing the characteristics of exosomes derived from glia-like cells (hereinafter referred to as "ghMSCs") pretreated with the peptide of SEQ ID NO: 1 according to one embodiment of the present invention (Example 1; Pep-EV(N)). FIGS. 1a, 1b, 1c, 1d, and 1e show the results of comparing the exosome production per cell between exosomes derived from glia-like cells (hereinafter referred to as "ghMSCs") pretreated with the peptide of SEQ ID NO: 1 according to one embodiment of the present invention (Example 1; Pep-EV(N)(F)) and exosomes derived from ghMSCs not pretreated with the peptide (Comparative Example 1; Natural-EV(N)(F)). In FIGS. 1a, 1b, 1c, 1d, and 1e, "(N)" denotes exosomes obtained from cells cultured in Neurobasal-A medium, and "(F)" denotes exosomes obtained from cells cultured in α-MEM medium containing 10% exosome-depleted FBS. FIG. 1f shows the results of Western blot analysis performed to confirm the expression of the exosomal positive markers CD9 and CD63 in Pep-EV(N) of Example 1. FIG. 1g shows transmission electron microscope (TEM) images illustrating the morphology of Pep-EV(N) of Example 1 and Natural-EV(N) of Comparative Example 1.

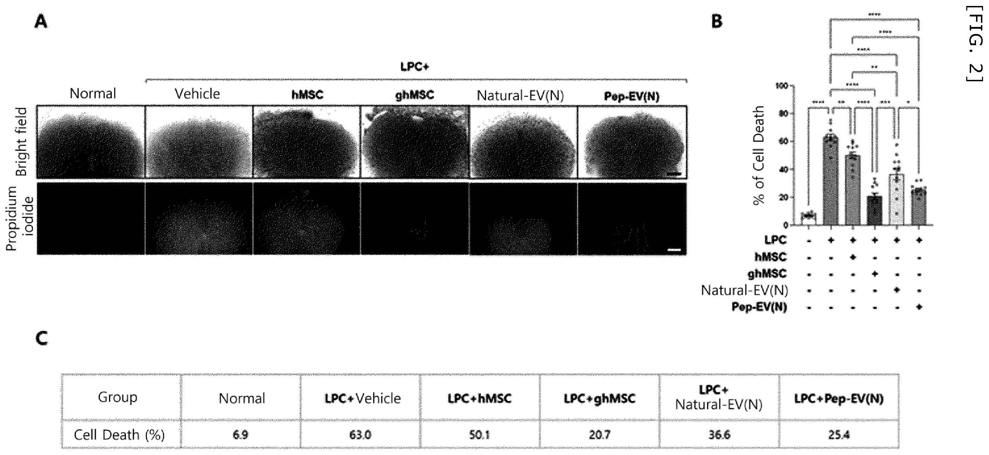

FIG. 2 shows the anti-apoptotic effect of ghMSC-derived exosomes pretreated with the peptide of SEQ ID NO: 1 according to one embodiment of the present invention in a nerve injury model. FIG. 2A shows fluorescence microscopic images obtained after treating the nerve injury model with exosomes isolated from ghMSCs and observing apoptosis seven days later. FIG. 2B is a graph showing quantitative analysis of the apoptosis images of FIG. 2A. FIG. 2C is a table showing numerical values corresponding to the graph of FIG. 2B. In FIG. 2, 'Normal' indicates a 'normal group or control group'; 'LPC+Vehicle' indicates a 'nerve injury-induced group or negative control group'; 'LPC+hMSC' indicates an 'hMSC (human mesenchymal stem cell)-treated group'; 'LPC+ghMSC' indicates a 'ghMSC-treated group or positive control group'; 'LPC+Natural-EV(N)' indicates a group treated with ghMSC-derived exosomes not pretreated with the peptide of Comparative Example 1; and "LPC+Pep-EV(N)" indicates a group treated with ghMSC-derived exosomes pretreated with the peptide of Example 1.

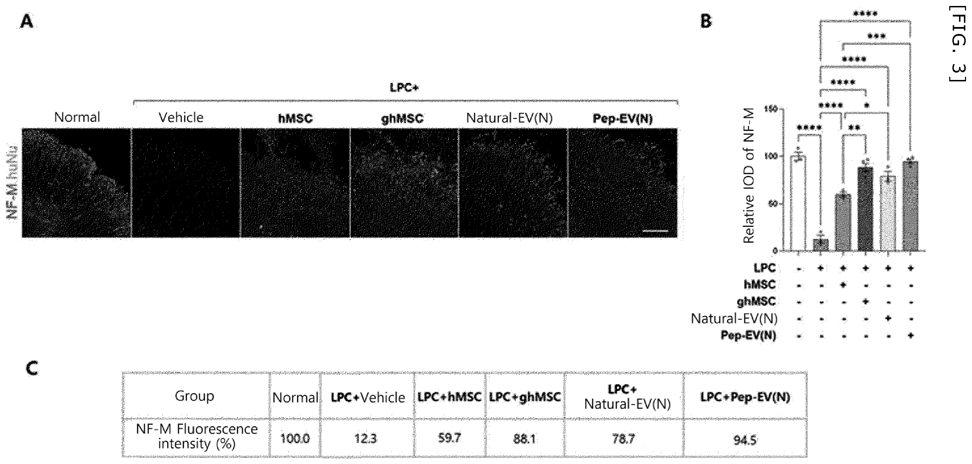

FIG. 3 shows the neural regenerative effect of ghMSC-derived exosomes pretreated with the peptide of SEQ ID NO: 1 according to one embodiment of the present invention in a nerve injury model. FIG. 3A shows fluorescence microscopic images obtained after treating the nerve injury model with exosomes isolated from ghMSCs and observing NF-M staining seven days later. FIG. 3B is a graph showing quantitative analysis of the NF-M fluorescence intensity shown in FIG. 3A. FIG. 3C is a table showing numerical values corresponding to the graph of FIG. 3B. In FIG. 3, 'Normal' indicates a 'normal group or control group'; 'LPC+Vehicle' indicates a 'nerve injury-induced group or negative control group'; 'LPC+hMSC' indicates an 'hMSC (human mesenchymal stem cell)-treated group'; 'LPC+ghMSC' indicates a 'ghMSC-treated group or positive control group'; 'LPC+Natural-EV(N)' indicates a 'group treated with ghMSC-derived exosomes not pretreated with the peptide' of Comparative Example 1; and 'LPC+Pep-EV(N)' indicates a 'group treated with ghMSC-derived exosomes pretreated with the peptide' of Example 1.

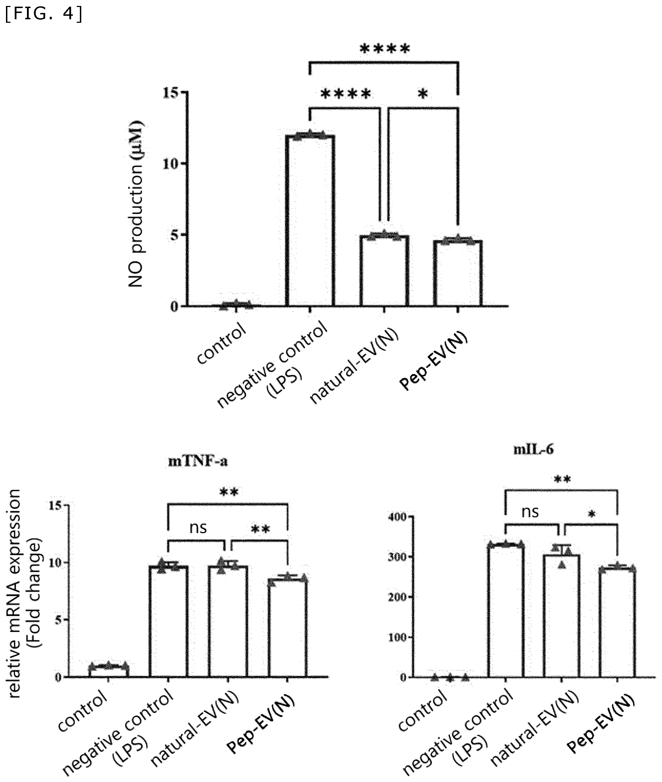

FIG. 4 is a graph showing the inhibitory effect of ghMSC-derived exosomes pretreated with the peptide of SEQ ID NO: 1 according to one embodiment of the present invention on neuroinflammation in a neuroinflammation model, wherein NO production, TNF-α expression, and IL-6 expression in the Example 1-treated group are compared with those in the control group, the negative control group (LPS), and the Comparative Example 1-treated group.

### BEST MODE FOR CARRYING OUT THE INVENTION

The present inventors previously obtained glia-like human mesenchymal stem cells (ghMSCs) exhibiting enhanced paracrine activity by inducing the differentiation of late-passage human mesenchymal stem cells (hMSCs), which have low neural functional recovery efficacy, into glia-like cells.

However, the ghMSCs were limited in that they did not overcome the disadvantages associated with conventional cell therapeutics, including difficulties in securing and maintaining therapeutically effective quantities of cells, immune rejection, and the risk of tumorigenesis.

Accordingly, the present inventors sought to develop extracellular vesicles possessing the intrinsic efficacy of the ghMSCs. However, extracellular vesicles isolated from the ghMSCs using conventional methods exhibited significantly reduced neuroprotective and neuroregenerative effects as compared with the ghMSCs themselves.

As a result of extensive studies conducted to solve the above-described problems, the present inventors specifically confirmed that extracellular vesicles isolated from ghMSCs pretreated with a human protein-derived peptide possess neuroprotective and neuroregenerative efficacy comparable to or greater than that of the ghMSCs, thereby completing the present invention.

Hereinafter, the present invention will be described in detail.

One aspect of the present invention relates to a pharmaceutical composition for preventing or treating a neurological disease caused by nerve injury, comprising an extracellular vesicle derived from a glia-like cell (ghMSC) differentiated from a human mesenchymal stem cell (hMSC) as an active ingredient, wherein the glia-like cell is pretreated with a peptide comprising the amino acid sequence of SEQ ID NO: 1.

In connection with the above aspect, the present invention also relates to a method for preventing, ameliorating, or treating a neurological disease caused by nerve injury, comprising administering, to a subject in need thereof, an effective amount of a composition comprising, as an active ingredient, extracellular vesicles derived from a glia-like cell (ghMSC) differentiated from human mesenchymal stem cell (hMSC), wherein the glia-like cell is pretreated with a peptide comprising the amino acid sequence of SEQ ID NO: 1.

In connection with the above aspect, the present invention also relates to a use of a composition comprising, as an active ingredient, extracellular vesicles derived from a glia-like cell (ghMSC) differentiated from a human mesenchymal stem cell (hMSC), wherein the glia-like cells are pretreated with a peptide comprising the amino acid sequence of SEQ ID NO: 1, for the manufacture of a medicament for preventing, ameliorating, or treating a neurological disease caused by nerve injury.

In connection with the above aspect, the present invention also relates to a composition for use in preventing, ameliorating, or treating neurological disease caused by nerve injury, comprising, as an active ingredient, extracellular vesicles derived from a glia-like cell (ghMSC) differentiated from a human mesenchymal stem cell (hMSC), wherein the glia-like cells are pretreated with a peptide comprising the amino acid sequence of SEQ ID NO: 1.

As used herein, the term "stem cell" refers to an undifferentiated cell at a stage prior to differentiation into tissue-forming cells and collectively encompasses cells having the ability to differentiate into specific cell types under particular differentiation stimuli or conditions. Unlike differentiated cells in which cell division has ceased, stem cells are characterized by the ability to self-renew through cell division and by plasticity, enabling differentiation into various cell types depending on the nature of the differentiation stimulus.

As used herein, the term "mesenchymal stem cell" refers to a multipotent stem cell capable of differentiating into adipocytes, osteocytes, chondrocytes, myocytes, neurons, and cardiomyocytes. The mesenchymal stem cells used in the present invention are not particularly limited, but are preferably bone marrow-derived mesenchymal stem cells.

Specifically, the bone marrow-derived mesenchymal stem cells of the present invention may be identified based on their spindle-shaped morphology and the expression profile of characteristic cell surface markers, including CD73(+), CD90(+), CD105(+), CD34(-), and CD45(-). Such cells also possess immunomodulatory functions in addition to multipotency.

In one embodiment, the peptide comprising the amino acid sequence of SEQ ID NO: 1 may be a peptide derived from the Noxa protein.

The Noxa protein may induce apoptosis by binding to and inhibiting Mcl1 and Bcl2A1 through its BH3 (Bcl-2 homology 3) domain, thereby activating BAX and BAK proteins, promoting the release of cytochrome c into the cytoplasm, and activating the caspase system.

In the present invention, the peptide may be prepared by direct chemical synthesis using solid phase peptide synthesis (SPPS), synthesis using an automated peptide synthesizer, or expression of a nucleotide sequence encoding the peptide after insertion thereof into a vector, although the present invention is not limited thereto.

In one embodiment, the peptide may have at least about 60%, at least about 70%, at least about 80%, at least about 90%, at least about 95%, at least about 98%, or at least about 99% sequence homology with a peptide consisting of the amino acid sequence of SEQ ID NO: 1.

As used herein, the term "glia-like cell (ghMSC; glia-like human mesenchymal stem cell)" refers to a late-passage mesenchymal stem cell differentiated from a human mesenchymal stem cell, particularly a bone marrow-derived mesenchymal stem cell, having enhanced neuroprotective and neuroregenerative efficacy due to its ability to secrete large amounts of glial growth factors.

The glia-like cells may be obtained, for example, by a method comprising primary culturing human mesenchymal stem cells in a medium comprising β-mercaptoethanol; secondary culturing the primary culture in a medium comprising all-trans-retinoic acid; and tertiary culturing the secondary culture in a medium comprising human basic fibroblast growth factor (hbFGF), human platelet-derived growth factor-AA (hPDGF-AA), forskolin, and heregulin-β1 (HRG-β1), thereby inducing differentiation into glia-like cells, although the present invention is not limited thereto.

In the primary culturing, β-mercaptoethanol may be present in the primary culture medium at a concentration of 0.05 mM to 20 mM, 0.1 mM to 10 mM, 0.2 mM to 5 mM, or 0.5 mM to 2 mM. In the secondary culturing, all-trans-retinoic acid may be present in the secondary culture medium at a concentration of 0.01 µg/mL to 2 µg/mL, 0.05 µg/mL to 1 µg/mL, or 0.1 µg/mL to 0.5 µg/mL. In the tertiary culturing, human basic fibroblast growth factor may be present in the tertiary culture medium at a concentration of 0.01 ng/mL to 1 ng/mL, 0.1 ng/mL to 100 ng/mL, 1ng/mL to 50ng/mL or 5 ng/mL to 20 ng/mL; human platelet-derived growth factor-AA may be present at a concentration of 0.1 ng/mL to 100 ng/mL, 0.5 ng/mL to 20 ng/mL, or 1 ng/mL to 10 ng/mL; forskolin may be present at a concentration of 0.5 µM to 100 µM, 1 µM to 50 µM, or 5 µM to 20 µM; and heregulin-β1 may be present at a concentration of 1 ng/mL to 1 µg/mL, 10 ng/mL to 500 ng/mL, or 50 ng/mL to 300 ng/mL. However, the present invention is not limited thereto. In addition, the medium composition may further comprise components such as DMEM, FBS, and the like, which are conventionally used to provide nutrients for cell culture, as well as antimicrobial and/or antiviral agents such as a penicillin/streptomycin mixture, although the present invention is not limited thereto.

As used herein, the term "extracellular vesicle" refers to a lipid bilayer vesicle having a diameter in the range of about 30 nm to about 1,000 nm and secreted into the extracellular environment through fusion of multivesicular bodies with the plasma membrane in various cell types.

In one embodiment, the extracellular vesicle is obtained by a method comprising the following steps a) differentiating a human bone marrow-derived mesenchymal stem cell to obtain a glia-like cell; b) culturing the glia-like cell in a medium composition comprising a peptide comprising the amino acid sequence of SEQ ID NO: 1; and c) collecting the medium composition of step b) comprising the extracellular vesicle and removing the stem cells through centrifugation.

In one embodiment, the concentration of the peptide contained in the medium composition may be 0.1 µM to 5.0 µM, 0.1 µM to 4.5 µM, 0.1 µM to 4.0 µM, 0.1 µM to 3.5 µM, 0.1 µM to 3.0 µM, 0.1 µM to 2.5 µM, 0.1 µM to 2.0 µM, 0.1 µM to 1.5 µM, 0.5 µM to 5.0 µM, 0.5 µM to 4.5 µM, 0.5 µM to 4.0 µM, 0.5 µM to 3.5 µM, 0.5 µM to 3.0 µM, 0.5 µM to 2.5 µM, 0.5 µM to 2.0 µM, or 0.5 µM to 1.5 µM, for example, 0.5 µM to 1.5 µM, although the present invention is not limited thereto.

In one embodiment, the medium composition of step b) may further comprise, as a cell culture medium composition for culturing stem cells, a mixture for in vitro cell growth and proliferation comprising components essential for cell growth and proliferation, such as sugars, amino acids, various nutrients, minerals, and the like.

Additional components that may be further included in the medium composition may include, for example, glycerin, L-alanine, L-arginine hydrochloride, L-cysteine hydrochloride monohydrate, L-glutamine, L-histidine hydrochloride monohydrate, L-lysine hydrochloride, L-methionine, L-proline, L-serine, L-threonine, L-valine, L-asparagine monohydrate, L-aspartic acid, L-cystine 2HCl, L-glutamic acid, L-isoleucine, L-leucine, L-phenylalanine, L-tryptophan, L-tyrosine disodium salt dihydrate, i-inositol, thiamine hydrochloride, niacinamide, pyridoxine hydrochloride, biotin, calcium D-pantothenate, folic acid, riboflavin, vitamin B₁₂, sodium chloride (NaCl), sodium bicarbonate (NaHCO₃), potassium chloride (KCl), calcium chloride (CaCl₂), sodium phosphate monohydrate (NaH₂PO₄·H₂O), copper sulfate pentahydrate (CuSO₄·5H₂O), ferrous sulfate heptahydrate (FeSO₄·7H₂O), magnesium chloride (anhydrous), magnesium sulfate (MgSO₄), disodium hydrogen phosphate (Na₂HPO₄), zinc sulfate heptahydrate (ZnSO₄·7H₂O), D-glucose (dextrose), sodium pyruvate, hypoxanthine Na, linolenic acid, lipoic acid, putrescine 2HCl, and thymidine, but is not limited thereto.

The medium composition according to the present invention may be artificially prepared and used, or may be purchased and used from commercially available products. Examples of commercially available culture media include, but are not limited to, IMDM (Iscove's Modified Dulbecco's Medium), α-MEM (Alpha Modification of Eagle's Medium), F12 (Nutrient Mixture F-12), and DMEM/F12 (Dulbecco's Modified Eagle Medium: Nutrient Mixture F-12).

In one embodiment, the "culturing" may be orbital shaking culture of the ghMSC in a state in which the ghMSC is floating as a single cell in the medium composition comprising the peptide.

As used herein, the term "orbital shaking culture" may refer to culturing cells at a constant rotational speed by placing a flask on a platform rotating in a horizontal circular orbit having a constant radius, but is not limited thereto.

In one embodiment, the orbital shaking culture may be performed using an orbital shaker.

In the step c), the stem cells are removed from the medium composition collected in step b) through a plurality of centrifugation procedures.

In one Example of the present invention, it was specifically confirmed that extracellular vesicles derived from ghMSCs pretreated with a peptide comprising the amino acid sequence of SEQ ID NO:1 exhibited an exosome production amount per cell increased by at least 4-fold, preferably at least 10-fold, as compared with extracellular vesicles derived from ghMSCs not pretreated with the peptide.

In one embodiment, the extracellular vesicles of the present invention have an average diameter of 50 to 300 nm, more specifically 100 to 250 nm, more specifically 150 to 220 nm, more specifically 180 to 200 nm, and more specifically 185 to 195 nm. Extracellular vesicles having such a fine diameter range are referred to as exosomes.

In one embodiment, the composition of the present invention may comprise the extracellular vesicles at a concentration of 10⁶ to 10¹⁴ particles/mL, preferably 10⁸ to 10¹⁴ particles/mL, more preferably 10⁸ to 10¹² particles/mL, more preferably 10¹⁰ to 10¹² particles/mL, and more preferably 10¹¹ to 10¹² particles/mL.

The composition of the present invention exhibits neuroprotective activity and neuroregenerative activity and thus may be used for prevention or treatment of neurological diseases caused by various nerve injuries.

In one embodiment, the term "nerve injury" may refer to injury to nerve tissue or nerve cells, including axonal degeneration or Wallerian degeneration. Further, depending on the degree of injury, the nerve injury may include, in peripheral nerves, neuropraxia without Wallerian degeneration, axonotmesis accompanied by Wallerian degeneration, or neurotmesis in which axonal continuity is disrupted.

In one embodiment, the "neurological disease caused by nerve injury" may be a neurological disease caused by nerve injury of the central nervous system or nerve injury of the peripheral nervous system, preferably a neurological disease caused by nerve injury of the peripheral nervous system, and may be accompanied or not accompanied by symptoms such as paralysis, convulsion, seizure, cognitive impairment, language impairment, memory impairment, abnormal behavior, emotional dysregulation, dizziness, vomiting, gait disturbance, hormonal abnormality, balance impairment, pain, sensory abnormality, motor dysfunction, numbness, cold sensation, or burning sensation depending on the site and severity of the injury.

For example, in the case of neurological diseases caused by nerve injury of the central nervous system, symptoms such as paralysis, convulsion, seizure, cognitive impairment, language impairment, memory impairment, abnormal behavior, emotional dysregulation, dizziness, vomiting, gait disturbance, hormonal abnormality, and balance impairment may be predominantly accompanied. In the case of neurological diseases caused by nerve injury of the peripheral nervous system, symptoms such as pain, sensory abnormality, motor dysfunction, numbness, cold sensation, and burning sensation may be predominantly accompanied. The central nervous system is a nervous system including the brain and spinal cord, and injury to the spinal cord due to trauma or disease may prevent normal neural transmission between the brain and the body, thereby resulting in motor or sensory paralysis. The peripheral nervous system is a nervous system including 12 pairs of cranial nerves, 31 pairs of spinal nerves, and autonomic nerves, and may cause symptoms such as facial paralysis due to facial nerve injury, blindness due to optic nerve injury, eyelid paralysis due to oculomotor nerve injury, hearing loss or vertigo due to auditory nerve injury, depending on the site of injury.

In one embodiment, the neurological disease caused by nerve injury of the central nervous system may be any one selected from the group consisting of organic diseases and functional disorders of the central nervous system, epilepsy, multiple sclerosis, amyotrophic lateral sclerosis, Alzheimer's disease, Lewy body dementia, Huntington's disease, Parkinson's disease, schizophrenia, traumatic brain injury, stroke, Pick's disease, Creutzfeldt-Jakob disease, progressive supranuclear palsy, multiple system atrophy, corticobasal degeneration, spinocerebellar degeneration, cerebellar atrophy, post-traumatic stress disorder, amnesia, vascular dementia, and cerebral infarction. As used herein, the "organic diseases and functional disorders of the central nervous system" may refer to diseases exhibiting symptoms such as paralysis, convulsion, seizure, cognitive impairment, language impairment, memory impairment, abnormal behavior, emotional dysregulation, dizziness, vomiting, gait disturbance, hormonal abnormality, balance impairment, and pain due to nerve injury of the central nervous system. In certain embodiments, the neurological disease caused by nerve injury of the central nervous system may be associated with central nervous system excitotoxicity. For example, the neurological disease may be epilepsy, multiple sclerosis, amyotrophic lateral sclerosis, Alzheimer's disease, Lewy body dementia, Huntington's disease, Parkinson's disease, schizophrenia, traumatic brain injury, stroke, or the like, and may further include other degenerative brain diseases associated with central nervous system excitotoxicity.

In one embodiment, the neurological disease caused by injury to the peripheral nervous system may be any one selected from the group consisting of peripheral neuropathy, diabetic neuropathy, peripheral neuropathic pain, chemotherapy-induced peripheral neuropathy, complex regional pain syndrome, optic neuropathy, mononeuropathy, multiple mononeuropathy (mononeuritis multiplex), polyneuropathy, Guillain-Barré syndrome (acute inflammatory demyelinating polyneuropathy), chronic inflammatory demyelinating polyneuropathy, hereditary neuropathy, plexopathy, glaucoma, macular degeneration, amyotrophic lateral sclerosis (ALS; Lou Gehrig's disease), progressive muscular atrophy, progressive bulbar palsy, poliomyelitis, post-polio syndrome, stiff-person syndrome, Isaacs syndrome, myasthenia gravis, neonatal myasthenia, botulism, Eaton-Lambert syndrome, thoracic outlet syndrome, Charcot-Marie-Tooth disease, and spinal muscular atrophy.

In one embodiment, the diabetic neuropathy may be polyneuropathy or focal neuropathy. In one embodiment, the polyneuropathy may be any one selected from the group consisting of hyperglycemic neuropathy, distal symmetric polyneuropathy, autonomic neuropathy, acute sensory neuropathy, acute painful sensory neuropathy, and chronic sensorimotor neuropathy. In one embodiment, the focal neuropathy may be any one selected from the group consisting of cranial neuropathy, truncal neuropathy, limb neuropathy, thoracolumbar radiculoneuropathy, and lumbosacral radiculoplexus neuropathy.

In one embodiment, the peripheral neuropathic pain may include pain caused by abnormalities or injury of the peripheral nervous system, trigeminal neuralgia, diabetes neuropathy pain, phantom limb pain, pain caused by viral infection, trauma, cancer, or alcoholism, pain occurring after chemotherapy, atypical facial pain, post-herpetic neuralgia, and neuropathic pain resulting from neurological disorders.

As used herein, unless otherwise indicated, the term "treatment" means reversing, alleviating, inhibiting the progression of, or preventing symptoms resulting from the nerve injury.

In one embodiment of the present invention, it was specifically confirmed that, when the glia-like cell-derived extracellular vesicles of the present invention were administered to an ex vivo nerve injury model, nerve tissues damaged by LPC (lysophosphatidylcholine) were significantly regenerated or restored.

In one embodiment, the pharmaceutical composition of the present invention may comprise the glia-like cell-derived extracellular vesicles alone, or may further comprise one or more pharmaceutically acceptable carriers, excipients, or diluents.

Examples of pharmaceutically acceptable carriers may include carriers for oral administration or carriers for parenteral administration. Oral administration carriers may include lactose, starch, cellulose derivatives, magnesium stearate, stearic acid, and the like. Parenteral administration carriers may include water, suitable oils, saline, aqueous glucose, glycols, and the like, and may further include stabilizers and preservatives. Suitable stabilizers include antioxidants such as sodium bisulfite, sodium sulfite, or ascorbic acid. Suitable preservatives include benzalkonium chloride, methyl- or propyl-paraben, and chlorobutanol. The pharmaceutical composition of the present invention may further comprise lubricants, wetting agents, sweetening agents, flavoring agents, emulsifying agents, suspending agents, and the like, in addition to the above-described components. Other pharmaceutically acceptable carriers may be referred to those described in the following document (Remington's Pharmaceutical Sciences, 19th ed., Mack Publishing Company, Easton, PA, 1995).

The composition of the present invention may be administered to mammals including humans by any method. For example, it may be administered orally or parenterally. The parenteral administration method may include, but is not limited to, intravenous administration, intramuscular administration, intra-arterial administration, intraosseous administration, intrathecal administration, suprachoroidal injection, transdermal administration, subcutaneous administration, intraperitoneal administration, intranasal administration, enteral administration, topical administration, sublingual administration, or rectal administration, and preferably may be intravenous administration.

According to the administration route as described above, the pharmaceutical composition of the present invention may be formulated into preparations for oral administration or parenteral administration.

In the case of a preparation for oral administration, the composition of the present invention may be formulated into powders, granules, tablets, pills, sugar-coated tablets, capsules, solutions, gels, syrups, slurries, suspensions, and the like by using methods known in the art. For example, oral preparations may be obtained by mixing the active ingredient with a solid excipient, pulverizing the mixture, adding suitable auxiliaries thereto, and then processing the resulting mixture into granular mixture, thereby obtaining tablets or sugar-coated tablets. Examples of suitable excipients may include sugars including lactose, dextrose, sucrose, sorbitol, mannitol, xylitol, erythritol, and maltitol; starches including corn starch, wheat starch, rice starch, and potato starch; celluloses including cellulose, methyl cellulose, sodium carboxymethyl cellulose, and hydroxypropyl methyl cellulose; and fillers such as gelatin and polyvinylpyrrolidone. In addition, where appropriate, cross-linked polyvinylpyrrolidone, agar, alginic acid, sodium alginate, or the like may be added as a disintegrant. Furthermore, the pharmaceutical composition of the present invention may further comprise anti-caking agents, lubricants, wetting agents, flavoring agents, emulsifying agents, preservatives, and the like.

In the case of a preparation for parenteral administration, it may be formulated into injections, ointments, creams, lotions, oils, gels, aerosols, and nasal inhalants by methods known in the art. These formulations are described in a generally known reference in pharmaceutical chemistry (Remington's Pharmaceutical Science, 15th Edition, 1975. Mack Publishing Company, Easton, Pennsylvania 18042, Chapter 87: Blaug, Seymour).

Preferably, the pharmaceutical composition of the present invention may be prepared in a form selected from the group consisting of an oral preparation, an injectable preparation, and an ointment preparation, and more preferably may be an injectable preparation.

The pharmaceutical composition of the present invention may provide a desirable neuroprotective and regenerative effect when comprising the stem cell-derived extracellular vesicles in an effective amount. As used herein, the term "effective amount" refers to an amount that exhibits a response greater than that of a negative control group, and preferably refers to an amount sufficient to alleviate or treat nerve injury. The glial cell-like cell-derived extracellular vesicles may be comprised at a concentration of 10⁶ to 10¹⁴ particles/mL based on the total content of the pharmaceutical composition, preferably at a concentration of 10⁸ to 10¹⁴ particles/mL, more preferably at a concentration of 10⁸ to 10¹² particles/mL, more preferably at a concentration of 10¹⁰ to 10¹² particles/mL, and still more preferably at a concentration of 10¹¹ to 10¹² particles/mL. In this regard, when the content of the glia-like cell-derived extracellular vesicles is less than the lower limit, cell viability may be excellent, but the therapeutic effect on nerve injury may not be exhibited to a desired extent. Conversely, when the content exceeds the upper limit, the therapeutic effect on nerve injury may not increase in proportion to the increase in concentration, or toxicity may occur. Meanwhile, as a result of ex vivo experiments, when the concentration of the glia-like cell-derived extracellular vesicles of the present invention was within the above range, a significant effect on the treatment of nerve injury was observed while side effects such as cytotoxicity were not observed. The effective amount of the glia-like cell-derived extracellular vesicles included in the pharmaceutical composition of the present invention may vary depending on the form in which the composition is formulated.

The total effective amount of the pharmaceutical composition of the present invention may be administered to a patient as a single dose, or may be administered by a fractionated treatment protocol in which multiple doses are administered over a prolonged period of time. The pharmaceutical composition of the present invention may vary in the content of the active ingredient depending on the severity of the disease.

A suitable dosage of the pharmaceutical composition of the present invention may be variously prescribed depending on factors such as formulation method, mode of administration, age, body weight, sex, pathological condition, food, administration time, administration route, excretion rate, and responsiveness of the patient. A preferred dosage of the pharmaceutical composition of the present invention is within the range of 0.001 to 100 mg/kg for an adult.

In another aspect, the present invention relates to a use of extracellular vesicles isolated from glia-like cells pretreated with a peptide comprising the amino acid sequence of SEQ ID NO: 1 for manufacture of a medicament for preventing or treating a neurological disease caused by nerve injury. As described above, the extracellular vesicles isolated from glia-like cells pretreated with a peptide comprising the amino acid sequence of SEQ ID NO: 1 may be used for preventing or treating a neurological disease caused by nerve injury.

In still another aspect, the present invention provides a method for preventing or treating a neurological disease caused by nerve injury, comprising administering to a mammal an effective amount of extracellular vesicles isolated from glia-like cells pretreated with a peptide comprising the amino acid sequence of SEQ ID NO: 1.

As used herein, the term "mammal" refers to a mammal that is a subject of treatment, observation, or experimentation, and preferably refers to a human.

As used herein, the term "effective amount" means an amount of an active ingredient or pharmaceutical composition effective to induce a biological or medical response in a tissue system, animal, or human, as contemplated by a researcher, veterinarian, physician, or other clinician, and includes an amount effective to induce alleviation of symptoms of the relevant disease or disorder. The effective amount and frequency of administration of the active ingredient of the present invention may vary depending on the desired effect. Accordingly, the optimum dosage to be administered may be readily determined by one skilled in the art and may be adjusted according to various factors including the type of disease, the severity of the disease, the content of the active ingredient and other ingredients contained in the composition, the type of formulation, and the age, body weight, general health condition, sex, and diet of the patient, the time of administration, the route of administration, the excretion rate of the composition, the treatment period, and drugs used concurrently therewith. In the method for alleviating or treating according to the present invention, for an adult, it is preferable to administer a composition comprising extracellular vesicles isolated from glia-like cells pretreated with a peptide comprising the amino acid sequence of SEQ ID NO: 1 at a dose of 0.001 g/kg to 10 g/kg when administered once to several times per day.

In the therapeutic method of the present invention, a composition comprising, as an active ingredient, extracellular vesicles isolated from glia-like cells pretreated with a peptide comprising the amino acid sequence of SEQ ID NO: 1 may be administered in a conventional manner through oral, rectal, intravenous, intra-arterial, intraperitoneal, intramuscular, intrasternal, transdermal, topical, intraocular, or intradermal routes.

In another aspect, the present invention relates to a method for preparing a stem cell-derived extracellular vesicle having improved nerve regeneration efficacy and yield, comprising the following steps:
a) differentiating human bone marrow-derived mesenchymal stem cells to obtain glia-like cells (ghMSCs);
b) culturing the glia-like cells in a medium composition comprising a peptide comprising the amino acid sequence of SEQ ID NO: 1; and
c) collecting the medium composition of step b) comprising the extracellular vesicles and removing the glia-like cells through centrifugation.

Specifically, step a) may comprise: primary culturing human bone marrow-derived mesenchymal stem cells in a medium comprising β-mercaptoethanol; secondary culturing the primary culture in a medium comprising tretinoin (all-trans-retinoic acid); and tertiary culturing the secondary culture in a medium comprising human basic fibroblast growth factor (hbFGF), human platelet derived growth factor AA (hPDGF-AA), forskolin, and heregulin-β1 (HRG-β1), thereby obtaining glia-like cells through differentiation.

According to the present invention, extracellular vesicles having nerve regeneration efficacy may be produced in high yield and high purity.

Hereinafter, the present invention will be described in detail with reference to Examples. However, the present invention is not limited by the following Examples.

### <Examples>

### Experimental method

### Culture of Human Mesenchymal Stem Cells (hMSCs)

Adult human mesenchymal stem cells (hMSCs) (STEMCELL Technologies Inc., Vancouver, Canada), isolated from normal human bone marrow, were cultured in low-glucose Dulbecco's Modified Eagle's Medium (DMEM, low glucose) supplemented with 10% FBS (Gibco, Waltham, MA, USA). The cells (passages 6-12) were subcultured under an atmosphere of 5% CO2 at 37°C to obtain late-passage hMSCs.

### Induction of Human Mesenchymal Stem Cells into Glia-Like Cell (ghMSC)

The obtained late-passage human mesenchymal stem cell (hMSC) were cultured for 24 hours after replacing the medium with a primary differentiation medium [DMEM-low glucose medium, 10% FBS, 1% penicillin-streptomycin mixture, and 1 mM β-mercaptoethanol (M3148, Sigma-Aldrich)]. Thereafter, the human mesenchymal stem cells were washed with PBS (phosphate-buffered saline) and cultured in a secondary differentiation medium [DMEM-low glucose medium, 10% FBS, 1% penicillin-streptomycin mixture, and 0.28 µg/mL tretinoin (all-trans-retinoic acid; R2625, Sigma-Aldrich)]. After 3 days, the mesenchymal stem cells were washed with PBS and cultured in a tertiary differentiation medium [DMEM-low glucose medium, 10% FBS, 1% penicillin-streptomycin mixture, 10 µM forskolin (F6886, Sigma-Aldrich), 10 ng/mL human basic fibroblast growth factor (hbFGF; 100-18B, PeproTech), 5 ng/mL human platelet derived growth factor-AA (hPDGF-AA; 100-13A, PeproTech), and 200 ng/mL heregulin-β1 (HRG-β1; 396-HB, R&D Systems)] for 8 days to obtain differentiated glia-like cells (ghMSCs). At this time, the tertiary differentiation medium was replaced every two days.

### Isolation of Extracellular Vesicles

### Example 1: Pep-EV (N)

The ghMSCs were seeded in a 100 mm dish (172931, Thermo Fisher Scientific) and cultured until the cells reached 80-90% confluence, after which the medium was replaced with Neurobasal-A (NB) medium (21103049, Gibco). After 18 hours from replacing the medium with the NB medium, the cells were brought into a floating state using TrypLE^{™} Select (12563029, Gibco). The suspended cells were centrifuged to obtain a pellet. The pellet was placed in a 50 mL conical tube (50050, SPL), and after adding a composition comprising a sucrose buffer and a peptide represented by SEQ ID NO: 1 (10 µM), the mixture was subjected to orbital shaking culture on an orbital shaker (60 rpm) for 30 minutes. Thereafter, the culture medium was collected and centrifuged at 300 × g for 3 minutes to remove stem cell debris. The resulting supernatant was centrifuged at 2,000 × g for 10 minutes and transferred to a new tube. The supernatant was then centrifuged again at 10,000 × g for 30 minutes. Subsequently, the resulting supernatant was centrifuged at 178,000 × g for 2 hours to obtain a pellet (extracellular vesicles, Pep-EV (N)).

### Example 2: Pep-EV (F)

Pep-EV (F) was prepared in the same manner as Pep-EV (N), except that α-MEM medium (12561072, Gibco) containing 10% exosome-depleted FBS was used instead of NB medium, and the cells were cultured for 48 hours instead of 18 hours to obtain extracellular vesicles (Pep-EV (F)).

### Comparative Example 1: Natural-EV (N)

The ghMSCs were seeded in a 100 mm dish (172931, Thermo Fisher Scientific) and cultured until the cells reached 80-90% confluence, after which the medium was replaced with Neurobasal-A (NB) medium (21103049, Gibco). Eighteen hours after replacement with the NB medium, the culture medium was collected and centrifuged at 300 × g for 3 minutes to remove stem cell debris. The resulting supernatant was centrifuged at 2,000 × g for 10 minutes and transferred to a new tube. The supernatant was then centrifuged again at 10,000 × g for 30 minutes. Subsequently, the resulting supernatant was centrifuged at 178,000 × g for 2 hours to obtain a pellet (extracellular vesicles, Natural-EV (N)).

### Comparative Example 2: Natural-EV (F)

Natural-EV (F) was prepared in the same manner as Natural-EV (N), except that α-MEM medium (12561072, Gibco) containing 10% exosome-depleted FBS was used instead of NB medium, and the cells were cultured for 48 hours instead of 18 hours to obtain extracellular vesicles (Natural-EV (F)).

### Analysis of Extracellular Vesicle Production Yield

The size, number, and concentration of EVs were measured by dynamic light scattering (DLS) using a nanoparticle tracking analyzer, ZetaView (Particle Metrix, Germany, TWIN PMX-220). The ZetaView measurements were performed under the following settings: focus, automatic; particles per frame, 150-200; frames per second, 30; camera sensitivity, 80; shutter speed, 100; scattering intensity, automatic; and temperature, 25°C.

### Confirmation of Exosome-Related Marker Expression - Western Blot Analysis

Western blot analysis was performed to confirm the protein expression profiles of the extracellular vesicles.

Specifically, cells were lysed using RIPA buffer (CBR002, LPS Solution) containing a protease inhibitor cocktail (87786, Invitrogen), and whole cell lysates (WCLs) were isolated. The WCLs and EVs (exosomes) were subjected to electrophoresis on 4-12% Bis-Tris Plus Gels (NW04125BOX, Invitrogen) and then transferred onto nitrocellulose (NC) membranes (IB23001, Invitrogen). Primary antibodies (1:1,000 dilution) were incubated overnight at 4°C, followed by washing three times with 1× TBST (TLP-118.1, TransLab). Thereafter, secondary antibodies were reacted for 2 hours at room temperature and washed with 1× TBST. All antibodies were diluted in 1× blocking buffer (TLP-115.1G, TransLab) before use, and signals were detected using Invitrogen^{™} iBright^{™} Imagers (CL-1000).

At this time, CD9 (ab263023, Abcam), CD63 (ab59479, Abcam), and CD81 (ab109201, Abcam), which are positive markers, were used as primary antibodies capable of targeting markers of the extracellular vesicles. In addition, each primary antibody was detected using a species-appropriate secondary antibody according to the information provided by the manufacturer.

### Confirmation of Exosome Morphology - Transmission Electron Microscopy Analysis

Exosomes were adsorbed onto copper grids 300-mesh formvar/carbon-coated (FCF300-CU, Electron Microscopy Sciences), negatively stained using 1% phosphotungstic acid hydrate (Sigma, P4006), and the morphology of the exosomes was observed using a transmission electron microscope (TEM; Hitachi, HT7800).

### Organotypic Spinal Cord Slice Culture

### Preparation of Nerve Injury Model

Spinal cords were isolated from 16-day-old rats and sectioned into slices having a thickness of 350 µm using a chopper. The spinal cord slices were cultured in a medium comprising 50% MEM + HEPES mixture, 25% horse serum, 25% HBSS (Hank's Balanced Salt Solution), 6.5 mg/mL glucose, and 1% penicillin/streptomycin. After culturing for 7 days, the spinal cord slices were treated with a culture medium containing 0.5 mg/mL LPC (lysophosphatidylcholine) for 17 hours to induce demyelination and nerve injury, thereby preparing a nerve injury model.

### PI (Propidium Iodide) Uptake Analysis and Image Analysis

Prior to exposure to LPC, the spinal cord slice tissues were treated with PI solution (5 µg/mL), and the basal fluorescence intensity (F₀) was recorded using a rhodamine filter of a fluorescence microscope. Thereafter, following exposure to LPC for 17 hours, PI solution (5 µg/mL) was applied, and the PI uptake intensity (Fₜ) was measured. Some spinal cord slice tissues were subjected to evaluation of cell death by replacing the medium with PBS and storing under refrigeration, followed by incubation at 4°C for 24 hours to induce cell death. Thereafter, PI solution (5 µg/mL) was applied, and the final PI uptake intensity (F_{fin}) was measured. For image evaluation, images were converted to an 8-bit format using ImageJ, and Gray-value mean intensity values were measured. The percentage of cell death was evaluated according to the following equation. [Equation 1] 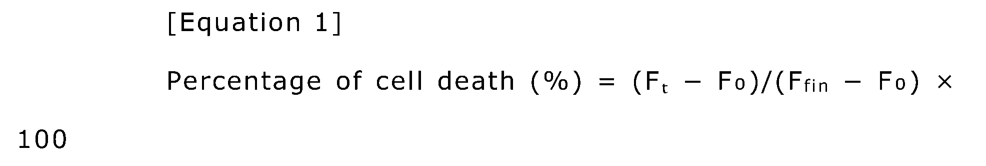 Percentage of cell death (%) = (Ft - F0)/(Ffin - F0) × 100

(F₀: basal fluorescence intensity, Fₜ: fluorescence intensity after exposure to LPC, F_{fin}: the final fluorescence intensity when all cells are dead)

### Evaluation of Neuroprotective Effects Following Treatment with ghMSC-Derived Exosomes in a Nerve Injury Model

The nerve injury model was treated with hMSC (human bone marrow-derived mesenchymal stem cell), ghMSC (glia-like cell, positive control), Natural-EV (N) (Comparative Example 1), and Pep-EV (N) (Example 1), respectively, followed by culture for 7 days. At this time, the cells (hMSC and ghMSC) were administered at a concentration of 3×10⁴ cells/1.5 µL/slice, and the exosomes (Natural-EV (N) and Pep-EV (N)) were administered at a concentration of 6×10⁷ particles/6 µL/slice. After the culture period, PI solution (5 µg/mL) was applied, and the spinal cord tissues were observed under a fluorescence microscope to compare the fluorescence intensity associated with cell death.

### Evaluation of Nerve Regeneration Effects Following Treatment with ghMSC-Derived Exosomes in a Nerve Injury Model

The nerve injury model was treated with hMSC (human bone marrow-derived mesenchymal stem cell), ghMSCs (glia-like cell), Natural-EV (N) (Comparative Example 1), and Pep-EV (N) (Example 1), respectively, followed by culture for 7 days. At this time, the cell (hMSC and ghMSC) were administered at a concentration of 3×10⁴ cells/1.5 µL/slice, and the exosomes (Natural-EV (N) and Pep-EV (N)) were administered at a concentration of 6×10⁷ particles/6 µL/slice. The cultured spinal cord tissues were fixed with 4% paraformaldehyde and then stained with NF-M (neurofilament medium polypeptide) and huNu (human nuclei). Thereafter, the degree of NF-M staining was observed under a fluorescence microscope, and the degree of nerve regeneration in the spinal cord tissues was compared based on fluorescence intensity.

### Evaluation of Therapeutic Efficacy Against Neuroinflammation Using BV2 Microglial Cells

BV2 microglial cells were seeded in a 24-well culture plate at 2×10⁵ cells/well and cultured for 24 hours. Thereafter, Natural-EV (N) (Comparative Example 1) and Pep-EV (N) (Example 1) were each mixed with DMEM-low glucose and used to pretreat the cells at a concentration of 1×10⁸ particles/mL for 6 hours. Subsequently, 100 ng/mL of LPS was added to each pretreated group and incubated for 24 hours. After 24 hours, the culture supernatants from each group were collected and centrifuged at 3,000 rpm for 1 minute to remove cellular debris, thereby obtaining the supernatants.

To measure nitric oxide production in BV2 cells in which inflammation had been induced by LPS, a Griess reagent was prepared by mixing 5% phosphoric acid/1% sulfanilamide and 0.1% N-(1-naphthyl)ethylenediamine dihydrochloride at a ratio of 1:1 (w/w). Thereafter, a mixture of 100 µL of the Griess reagent and 100 µL of the cell culture supernatant was added to a 96-well culture plate and incubated at room temperature for 10 minutes. The nitric oxide concentration was quantified by measuring absorbance at 540 nm using a Bio-RAD x-Mark^{™} spectrophotometer.

To determine the expression levels of TNF-α and IL-6 in BV2 cells in which inflammation had been induced by LPS, RT-qPCR was performed using mTNF-α and mIL-6 primers, and gene expression levels were quantified.

### Experimental Results

### Test Example 1: Characterization of Exosomes

### 1-1: Comparison of Exosome Production Yield Per Cell

The production yields of exosomes according to the above Examples and Comparative Examples were numerically converted and are shown in Table 1 below.

**[Table 1]**

| Group | Cell line | Harvested cell number | Peptide concent ration | Prep volume | EV particle no./ml | EV particle no./cell |
|---|---|---|---|---|---|---|
| Example 1 | ghMS C | 3.4E+6 | 10µM | 0.2 ml | 3.8E+11 | 2.235E+ 4 |
| Example 2 | ghMS C | 3.2E+6 | 10µM | 0.2 ml | 2.6E+11 | 1.625E+ 4 |
| Comparative example 1 | ghMS C | 3.4E+6 | - | 0.2 ml | 3.2E+10 | 1.882E+ 3 |
| Comparative example 2 | ghMS C | 3.2E+6 | - | 0.2 ml | 6.2E+10 | 3.750E+ 3 |

Referring to Table 1 above, Comparative Example 1 showed an exosome production yield of about 1,882 exosomes per cell, whereas Example 1 showed an exosome production yield of about 22,350 exosomes per cell, indicating that the production yield per cell was increased by approximately 11-fold. In addition, Comparative Example 2 showed an exosome production yield of about 3,750 exosomes per cell, whereas Example 2 showed an exosome production yield of about 16,250 exosomes per cell, indicating that the production yield per cell was increased by more than about 4-fold. Further, as a result of characterization of Example 1, it was confirmed that Example 1 exhibited the same characteristics as Comparative Example 1, which was produced without peptide treatment, in terms of both diameter and protein expression.

These results indicate that pretreatment of ghMSCs with the peptide of the present invention can significantly improve exosome production yield per cell (FIGS. 1a, 1b, 1c, 1d and 1e).

### 1-2: Confirmation of Exosome-Related Marker Expression

As a result of western blotting analysis of the exosomes according to Example 1, expression of CD9 and CD63, which are positive markers of exosomes, was observed, thereby specifically confirming that the isolated material was exosomes (FIG. 1f).

### 1-3: Morphological Analysis of Exosomes

As a result of negative staining of the exosomes according to Example 1 and Comparative Example 1, it was confirmed that Example 1 and Comparative Example 1 exhibited the same pattern in terms of morphology (FIG. 1g).

### Test Example 2: Neuroprotective Effects of ghMSC-Derived Exosomes in a Nerve Injury Model

The nerve injury model was treated with hMSC (human bone marrow-derived mesenchymal stem cell), ghMSC (glia-like cell, positive control), Natural-EV (N) (Comparative Example 1), and Pep-EV (N) (Example 1), respectively, followed by culture for 7 days, and cell death was then observed (FIG. 2 and Table 2).

**[Table 2]**

| Group | Normal | Negative control (LPC+Veh icle) | LPC+ hMSC | Positive control (LPC+gh MSC) | Compar ative example 1 | Example 1 |
|---|---|---|---|---|---|---|
| Cell death (%) | 6.9 | 63.0 | 50.1 | 20.7 | 36.6 | 25.4 |
| Relativ e ratio | - | 100 | 79.5 | 32.9 | 58.1 | 40.3 |
| Rate of change | - | - | 20.5 % decrease | 67.1% decrease | 41.9% decrease | 59.7% decrease |

Referring to FIG. 2 and Table 2, it was confirmed that the ghMSC-treated group (positive control) exhibited a 67.1% reduction in cell death as compared with the LPC+Vehicle group (nerve injury-induced group, negative control). In comparison, the Natural-EV (N)-treated group (Comparative Example 1) exhibited a 41.9% reduction in cell death as compared with the negative control group, whereas the Pep-EV (N)-treated group (Example 1) of the present invention exhibited a 59.7% reduction in cell death as compared with the negative control group, demonstrating that the neuroprotective effect was significantly improved compared with Comparative Example 1.

### Test Example 3: Nerve Regeneration Effects of ghMSC-Derived Exosomes in a Nerve Injury Model

The nerve injury model was treated with hMSCs (human bone marrow-derived mesenchymal stem cells), ghMSCs (glia-like cells, positive control), Natural-EV (N) (Comparative Example 1), and Pep-EV (N) (Example 1), respectively, followed by culture for 7 days. Thereafter, NF-M, a nerve regeneration marker, was stained and observed (FIG. 3 and Table 3).

**[Table 3]**

| Group | Normal | Negative control (LPC+Veh icle) | LPC+ hMSC | Positive control (LPC+gh MSC) | Comparative example 1 | Example 1 |
|---|---|---|---|---|---|---|
| NF-M Fluores cence intensity (%) | 100. 0 | 12.3 | 59.7 | 88.1 | 78.7 | 94.5 |
| Relative ratio to the negative control group | - | - | 4.85-fold | 7.16-fold | 6.40-fold | 7.68-fold |

Referring to FIG. 3 and Table 3, it was confirmed that the ghMSC-treated group (positive control) exhibited a 7.16-fold increase in nerve regeneration as compared with the LPC+Vehicle group (nerve injury-induced group, negative control). In comparison, the Natural-EV (N)-treated group (Comparative Example 1) exhibited only a 6.4-fold increase in nerve regeneration as compared with the negative control group, whereas the Pep-EV (N)-treated group (Example 1) of the present invention exhibited a 7.68-fold increase in nerve regeneration as compared with the negative control group, demonstrating that the nerve regeneration effect was significantly improved compared with Comparative Example 1. In particular, the Pep-EV (N)-treated group of Example 1 exhibited a nerve regeneration effect superior even to that of the ghMSC-treated group, which served as the positive control. These results indicate that pretreatment of ghMSCs with the peptide of the present invention enables the production of exosomes having excellent nerve regeneration effects. Specifically, it was confirmed that exosomes isolated from ghMSCs pretreated with the peptide of the present invention exhibited superior nerve regeneration effects compared with ghMSC-derived exosomes that were not pretreated with the peptide.

### Test Example 4: Anti-Inflammatory Effects of ghMSC-Derived Exosomes in a Neuroinflammation Model

The BV2 microglial cell-based neuroinflammation model was treated with Natural-EV (N) (Comparative Example 1) and Pep-EV (N) (Example 1), respectively, followed by culture for 6 hours. LPS was then added, and the cells were further cultured for 24 hours. Thereafter, nitric oxide (NO) production and the gene expression levels of the inflammatory factors TNF-α and IL-6 were evaluated (FIG. 4 and Table 4).

**[Table 4]**

| Group | Normal | Negative control (LPS+Vehi cle) | Comparative example (LPS+N atural-EV(N)) | Example 1 (LPS+Pep -EV(N)) |
|---|---|---|---|---|
| NO Production (µM) | 0.09 | 12.01 | 4.95 | 4.63 |
| TNF-α Expression Level (Relative to the Negative Control Group) | - | 100 | 100 | 86 |
| IL-6 Expression Level (Relative to the Negative Control Group) | - | 100 | 90 | 82 |

Referring to FIG. 4 and Table 4, both the Natural-EV (N)-treated group (Comparative Example 1) and the Pep-EV (N)-treated group (Example 1) exhibited significantly reduced NO production as compared with the negative control group. In addition, comparison of the expression levels of inflammatory factor genes in microglial cells showed that, in Comparative Example 1, the gene expression level of TNF-α was reduced by 10% and the gene expression level of IL-6 was not reduced, as compared with the negative control group. In contrast, in Example 1, the gene expression level of TNF-α was reduced by about 20% and the gene expression level of IL-6 was reduced by about 15%, as compared with the negative control group. Accordingly, it was confirmed that Example 1 exhibited a significantly improved neuroinflammation-inhibitory effect as compared with Comparative Example 1.

Although the present invention has been described with reference to the preferred embodiments described above, various modifications and variations may be made without departing from the spirit and scope of the invention. Further, the appended claims are intended to cover such modifications and variations as fall within the spirit of the present invention.

The present invention was made with support from the following research project.
[National Research and Development Project Supporting the Present Invention]
[Unique Project Number] 2710003628
[Project Number] 24A0203L1
[Ministry] Ministry of Science and ICT
[Project Management Agency] Korea Fund for Regenerative Medicine
[Research Program] Korea Fund for Regenerative Medicine Program
[Project Title] Advancement of an Endogenous Stem Cell Activation Platform Based on Immunomodulation and Enhanced Tissue Regeneration for the Treatment of Refractory Neuropathies, and Development of Core Technology for Delivery of Inductive Factors
[Performing Institution] Konkuk University Industrial Cooperation Corp
[Research Period] Apr. 1, 2024 - Dec. 31, 2024

## Claims

1. A pharmaceutical composition for preventing or treating a neurological disease caused by nerve injury, comprising an extracellular vesicle derived from a glia-like cell (ghMSC) differentiated from a human mesenchymal stem cell (hMSC) as an active ingredient,
wherein the glia-like cell is pretreated with a peptide comprising the amino acid sequence of SEQ ID NO: 1.

2. The composition according to claim 1,
wherein the mesenchymal stem cell is a bone marrow-derived mesenchymal stem cell.

3. The composition according to claim 1,
wherein the extracellular vesicle is obtained by a method comprising the following steps:
a) differentiating a human bone marrow-derived mesenchymal stem cell to obtain a glia-like cell;
b) culturing the glia-like cell in a medium composition comprising a peptide comprising the amino acid sequence of SEQ ID NO: 1; and
c) collecting the medium composition of step b) comprising the extracellular vesicle and removing the stem cells through centrifugation.

4. The composition according to claim 1,
wherein the extracellular vesicle has an average diameter of 50 to 300 nm.

5. The composition according to claim 1,
wherein the composition comprises the extracellular vesicle at a concentration of 10^6 to 10^14 particles/mL.

6. The composition according to claim 1,
wherein the neurological disease caused by nerve injury is a neurological disease caused by nerve injury of the central nervous system or nerve injury of the peripheral nervous system.

7. The composition according to claim 6,
wherein the neurological disease caused by nerve injury of the central nervous system is at least one selected from the group consisting of organic diseases and functional disorders of the central nervous system, epilepsy, multiple sclerosis, amyotrophic lateral sclerosis (ALS, Lou Gehrig's disease), Alzheimer's disease, Lewy body dementia, Huntington's disease, Parkinson's disease, schizophrenia, traumatic brain injury, stroke, Pick's disease, Creutzfeldt-Jakob disease, progressive supranuclear palsy, multiple system atrophy, corticobasal degeneration, spinocerebellar degeneration, cerebellar atrophy, post-traumatic stress disorder, amnesia, vascular dementia, and cerebral infarction.

8. The composition according to claim 6,
wherein the neurological disease caused by nerve injury of the peripheral nervous system is at least one selected from the group consisting of peripheral neuropathy, diabetic neuropathy, peripheral neuropathic pain, chemotherapy-induced peripheral neuropathy, complex regional pain syndrome, optic neuropathy, mononeuropathy, multiple mononeuropathy (mononeuritis multiplex), polyneuropathy, Guillain-Barré syndrome (acute inflammatory demyelinating polyneuropathy), chronic inflammatory demyelinating polyneuropathy, hereditary neuropathy, plexopathy, glaucoma, macular degeneration, amyotrophic lateral sclerosis (ALS, Lou Gehrig's disease), progressive muscular atrophy, progressive bulbar palsy, poliomyelitis, post-polio syndrome, stiff-person syndrome, Isaacs' syndrome, myasthenia gravis, neonatal myasthenia, botulism, Eaton-Lambert syndrome, thoracic outlet syndrome, Charcot-Marie-Tooth disease, and spinal muscular atrophy.

9. A method for preparing a stem cell-derived extracellular vesicle having improved nerve regeneration efficacy and yield, comprising the following steps:
a) differentiating a human bone marrow-derived mesenchymal stem cell to obtain a glia-like cell (ghMSC);
b) culturing the glia-like cell in a medium composition comprising a peptide comprising the amino acid sequence of SEQ ID NO: 1; and
c) collecting the medium composition of step b) comprising the extracellular vesicle and removing the glia-like cell through centrifugation.

10. A method for preventing, ameliorating, or treating a neurological disease caused by nerve injury, comprising administering, to a subject in need thereof, an effective amount of a composition comprising, as an active ingredient, an extracellular vesicle derived from a glia-like cell (ghMSC) differentiated from a human mesenchymal stem cell (hMSC),
wherein the glia-like cell is pretreated with a peptide comprising the amino acid sequence of SEQ ID NO: 1.

11. The method according to claim 10,
wherein the mesenchymal stem cell is a bone marrow-derived mesenchymal stem cell.

12. The method according to claim 10,
wherein the extracellular vesicle is obtained by a method comprising the following steps:
a) differentiating a human bone marrow-derived mesenchymal stem cell to obtain a glia-like cell;
b) culturing the glia-like cell in a medium composition comprising a peptide comprising the amino acid sequence of SEQ ID NO: 1; and
c) collecting the medium composition of step b) comprising the extracellular vesicle and removing the stem cells through centrifugation.

13. The method according to claim 10,
wherein the extracellular vesicle has an average diameter of 50 to 300 nm.

14. The method according to claim 10,
wherein the composition comprises the extracellular vesicle at a concentration of 10^6 to 10^14 particles/mL.

15. The method according to claim 10,
wherein the neurological disease caused by nerve injury is a neurological disease caused by nerve injury of the central nervous system or nerve injury of the peripheral nervous system.

16. The method according to claim 15,
wherein the neurological disease caused by nerve injury of the central nervous system is at least one selected from the group consisting of organic diseases and functional disorders of the central nervous system, epilepsy, multiple sclerosis, amyotrophic lateral sclerosis (ALS, Lou Gehrig's disease), Alzheimer's disease, Lewy body dementia, Huntington's disease, Parkinson's disease, schizophrenia, traumatic brain injury, stroke, Pick's disease, Creutzfeldt-Jakob disease, progressive supranuclear palsy, multiple system atrophy, corticobasal degeneration, spinocerebellar degeneration, cerebellar atrophy, post-traumatic stress disorder, amnesia, vascular dementia, and cerebral infarction.

17. The method according to claim 15,
wherein the neurological disease caused by nerve injury of the peripheral nervous system is at least one selected from the group consisting of peripheral neuropathy, diabetic neuropathy, peripheral neuropathic pain, chemotherapy-induced peripheral neuropathy, complex regional pain syndrome, optic neuropathy, mononeuropathy, multiple mononeuropathy (mononeuritis multiplex), polyneuropathy, Guillain-Barré syndrome (acute inflammatory demyelinating polyneuropathy), chronic inflammatory demyelinating polyneuropathy, hereditary neuropathy, plexopathy, glaucoma, macular degeneration, amyotrophic lateral sclerosis (ALS, Lou Gehrig's disease), progressive muscular atrophy, progressive bulbar palsy, poliomyelitis, post-polio syndrome, stiff-person syndrome, Isaacs' syndrome, myasthenia gravis, neonatal myasthenia, botulism, Eaton-Lambert syndrome, thoracic outlet syndrome, Charcot-Marie-Tooth disease, and spinal muscular atrophy.
